# EUROPÄISCHE PATENTANMELDUNG

(11) **EP 2 505 989 A1**
(43) Veröffentlichungstag der Anmeldung: **03.10.2012**
(21) Anmeldenummer: 12160675.0
(22) Anmeldetag: 22.03.2012
(51) Int. Cl.: G01N 21/64, A61B 1/00, A61B 1/06, A61B 1/04

(54) **Vorrichtung zur Fluoreszenzdiagnose**

(30) Priorität: 30.03.2011 DE 102011016138
(71) Anmelder: Karl Storz GmbH & Co. KG, 78532 Tuttlingen (DE)
(72) Erfinder: Fallert, Johannes, 78532 Tuttlingen (DE); Schwarz, Peter, 78532 Tuttlingen-Nendingen (DE); Göbel, Werner, 78224 Singen (DE); Ehrhardt, André, 78573 Wurmlingen (DE); Irion, Klaus-Martin, 78576 Emmingen-Liptingen (DE)
(74) Vertreter: Witte, Weller & Partner

(57) **Zusammenfassung**

Eine Vorrichtung (10) zur Fluoreszenzdiagnose weist ein Beleuchtungssystem (12) zum Beleuchten eines Zielgebiets (75), das eine Lichtquelle (16), die dazu ausgelegt ist, Weißlicht zu erzeugen, und einen Beleuchtungspfad (20) aufweist, und ein Beobachtungssystem (14) zum Beobachten des Zielgebiets (75) in einem Weißlichtmodus und in einem Fluoreszenzmodus auf, wobei das Beobachtungssystem (14) einen Beobachtungspfad (36) aufweist, der zumindest einen ersten Bildsensor (44) zum Empfangen eines Weißlichtbildes und zumindest einen zweiten Bildsensor (46) zum Empfangen eines Fluoreszenzbildes aufweist, wobei dem zweiten Bildsensor (46) im Beobachtungspfad (36) ein Beobachtungsspektralfilter (48) zugeordnet ist, dessen Filtercharakteristik (62) so ausgelegt ist, dass dem zweiten Bildsensor (46) Licht im Spektralbereich zu beobachtender Fluoreszenz zugespeist werden kann, während Licht in den Spektralbereichen außerhalb der Fluoreszenz abgeblockt wird. Im Beleuchtungspfad (20) ist sowohl im Weißlichtmodus als auch im Fluoreszenzmodus ein Beleuchtungsspektralfilter (60) angeordnet, dessen Filtercharakteristik (66) auf die Filtercharakteristik (62) des dem zweiten Bildsensor (46) zugeordneten Beobachtungsspektralfilters (48) so abgestimmt ist, dass das Zielgebiet (75) mit dem gesamten Spektralbereich des Weißlichts mit Ausnahme des Spektralbereichs der zu beobachtenden Fluoreszenz beleuchtet wird (Fig. 2).

## Beschreibung

Die Erfindung betrifft eine Vorrichtung zur Fluoreszenzdiagnose, mit einem Beleuchtungssystem zum Beleuchten eines Zielgebiets, das eine Lichtquelle, die dazu ausgelegt ist, Weißlicht zu erzeugen, und einen Beleuchtungspfad aufweist, und mit einem Beobachtungssystem zum Beobachten des Zielgebiets in einem Weißlichtmodus und in einem Fluoreszenzmodus, wobei das Beobachtungssystem einen Beobachtungspfad aufweist, der zumindest einen ersten Bildsensor zum Empfangen eines Weißlichtbildes und zumindest einen zweiten Bildsensor zum Empfangen eines Fluoreszenzbildes aufweist, wobei dem zweiten Bildsensor im Beobachtungspfad ein Beobachtungsspektralfilter zugeordnet ist, dessen Filtercharakteristik so ausgelegt ist, dass dem zweiten Bildsensor Licht im Spektralbereich zu beobachtender Fluoreszenz zugespeist werden kann, während Licht in den Spektralbereichen außerhalb der Fluoreszenz abgeblockt wird.

Eine solche Vorrichtung ist aus US 2003/0078477 A1 bekannt.

Eine erfindungsgemäße Vorrichtung zur Fluoreszenzdiagnose der eingangs genannten Art wird vorzugsweise zu medizinischen Diagnosezwecken verwendet, kann aber auch zu technischen Diagnosezwecken in industriellen oder wissenschaftlichen Anwendungen verwendet werden.

Im Rahmen der medizinischen Fluoreszenzdiagnose wird eine Vorrichtung der eingangs genannten Art zur Beurteilung des Zustands von biologischem Gewebe, beispielsweise allgemein zur Gewebedifferenzierung, insbesondere zur Tumorerkennung, aber auch zur Erkennung der Durchblutung und Vitalität verwendet. Mit einer Vorrichtung zur Fluoreszenzdiagnose der eingangs genannten Art kann die Fluoreszenzdiagnose insbesondere *in vivo* durchgeführt werden.

Bei der Fluoreszenzdiagnose wird eine im Zielgebiet vorhandene fluoreszierende Substanz mittels Licht zur Fluoreszenz angeregt. Dabei kann eine fluoreszierende Substanz ein Fluoreszenzfarbstoff, ein Pigment usw. sein, der zuvor in das Zielgebiet eingebracht wurde, beispielsweise durch Verabreichen der fluoreszierenden Substanz oder einer Vorstufe derselben an einen Patienten. Eine fluoreszierende Substanz kann jedoch auch eine bereits im Zielgebiet vorhandene Substanz sein, beispielsweise eine gewebespezifische Substanz, die durch die Fluoreszenzdiagnose zur Autofluoreszenz angeregt wird. Die vorliegende Erfindung kann beide Fälle umfassen. Für die nachfolgenden Erläuterungen wird von dem Fall ausgegangen, dass die fluoreszierende Substanz ein Fluoreszenzfarbstoff ist, der exogen (von außen) in das Zielgebiet, d.h. in das zu untersuchende Gewebeareal eingebracht wird.

Dazu wird dem zu untersuchenden Patienten der Fluoreszenzfarbstoff oder eine Ausgangsform dieses Fluoreszenzfarbstoffes verabreicht. In der Fluoreszenzdiagnose zur Gewebsuntersuchung wird dem Patienten als Ausgangssubstanz beispielsweise 5-Aminolävolinsäure verabreicht, aus der durch Hämbiosynthese intrazellulär der Fluoreszenzfarbstoff Protoporphyrin IX (PpIX) gebildet wird. Bei der Fluoreszenzdiagnose wird ausgenutzt, dass sich der Fluoreszenzfarbstoff, im vorstehenden Fall Protoporphyrin IX, in malignem Gewebe, beispielsweise Tumorgewebe, stärker anreichert als in gesundem Gewebe.

Der Fluoreszenzfarbstoff kann durch Beleuchtung mit Licht in einem Spektralbereich, in dem der Fluoreszenzfarbstoff absorbiert, zur Fluoreszenz angeregt werden. Beispielsweise der Fluoreszenzfarbstoff Protoporphyrin IX absorbiert am stärksten in einem Spektralbereich um 400 nm (Soret-Bande), also im violett-blauen sichtbaren Spektralbereich. Das Fluoreszenzlicht ist stets langwelliger als das Anregungslicht, und bei dem Fluoreszenzfarbstoff Protoporphyrin IX liegt die Fluoreszenzemission im sichtbaren Spektralbereich mit einem Maximum bei 635 nm und einem weiteren schwächeren Peak bei 705 nm, also im roten sichtbaren Spektralbereich. Die Intensität des emittierten Fluoreszenzlichts ist wegen des großen Stokes-Shifts von PpIX wesentlich geringer als die Intensität des reflektierten Anregungslichts.

Die eingangs genannte bekannte Vorrichtung zur Fluoreszenzdiagnose kann in zwei Betriebsarten betrieben werden, und zwar in einem Weißlichtmodus und in einem Fluoreszenzmodus. Im Fluoreszenzmodus wird die Lichtquelle, beispielsweise eine Quecksilberlampe oder eine Xenonlampe, so betrieben, dass sie Licht einer Wellenlänge im Spektralbereich von 380 nm bis 580 nm emittiert. Licht dieser Wellenlänge wird im Fluoreszenzmodus in das Zielgebiet gerichtet, wo es den Fluoreszenzfarbstoff Protoporphyrin IX zur Fluoreszenz anregt. Des Weiteren wird das in das Zielgebiet eingestrahlte Beleuchtungslicht im Zielgebiet reflektiert. Sowohl das reflektierte Licht als auch das Fluoreszenzlicht werden dann über den Beobachtungspfad zu zwei Bildsensoren geleitet, von denen der eine zum Empfangen eines Weißlichtbildes und der andere zum Empfangen eines Fluoreszenzbildes ausgelegt ist. Um zu vermeiden, dass das breitbandige reflektierte Beleuchtungslicht zu dem Bildsensor zum Empfangen des Fluoreszenzbildes gelangt, ist bei der bekannten Vorrichtung ein dichroitischer Spiegel vorgesehen, der als wellenlängenselektiver Strahlteiler wirkt, wobei der dichroitische Spiegel das breitbandige rückreflektierte Licht durchlässt und das schmalbandige Fluoreszenzlicht zu dem Bildsensor zum Empfangen des Fluoreszenzbildes richtet. Im Fluoreszenzmodus dieser bekannten Vorrichtung kann somit sowohl gleichzeitig ein Fluoreszenzbild als auch ein Weißlichtbild empfangen werden. Das empfangene Weißlichtbild ist jedoch aufgrund der Tatsache, dass im Fluoreszenzmodus das Beleuchtungslicht auf einem Wellenlängenbereich von 380 nm bis 580 nm begrenzt ist, nicht farbecht, weil Rot-Anteile des sichtbaren Lichts im Weißlichtbild fehlen. Das Fluoreszenzbild und das hinsichtlich der Farbtreue ungenügende Weißlichtbild werden auf einem Monitor mittels eines Bildverarbeitungssystems übereinanderliegend dargestellt.

Für ein farbtreues Weißlichtbild muss die bekannte Vorrichtung in den Weißlichtmodus umgeschaltet werden, wobei dann der dichroitische Spiegel aus dem Beobachtungspfad herausgeschwenkt ist. Hierdurch empfängt der Bildsensor zum Empfangen des Weißlichtbildes auch Anteile im roten Spektralbereich.

Nachteilig an dieser bekannten Vorrichtung ist es, dass es die Vorrichtung nicht ermöglicht, im Fluoreszenzmodus zusätzlich zum Fluoreszenzbild ein farbtreues Weißlichtbild zu erzeugen, und zum Erzeugen eines Weißlichtbildes die Vorrichtung in den Weißlichtmodus umgeschaltet werden muss. Hierdurch ist nicht nur die Handhabung der bekannten Vorrichtung aufwendig, sondern auch der konstruktive Aufwand aufgrund des Umschaltens der Lichtquelle und des Ein- und Ausschwenkens des dichroitischen Spiegels erhöht.

Eine weitere Vorrichtung zur Fluoreszenzdiagnose ist aus DE 10 2009 018 141 A1 bekannt. Diese bekannte Vorrichtung kann ebenfalls in einem Weißlichtmodus und in einem Fluoreszenzmodus betrieben werden, wobei bei dieser Vorrichtung jedoch nur ein Bildsensor vorhanden ist, der sowohl zum Empfangen eines Weißlichtbildes als auch zum Empfangen eines Fluoreszenzbildes verwendet wird.

Im Fluoreszenzmodus ist im Beleuchtungspfad ein Beleuchtungsspektralfilter angeordnet, der in einem schmalbandigen Anregungsspektralbereich zur Anregung der Fluoreszenz durchlässig und im Spektralbereich auf der langwelligen Seite außerhalb des Anregungsspektralbereichs im Wesentlichen undurchlässig ist. Dieses Beleuchtungsspektralfilter ist nur dann im Beleuchtungspfad angeordnet, wenn die Vorrichtung im Fluoreszenzmodus betrieben wird, während es im Weißlichtmodus aus dem Beleuchtungspfad entfernt ist. Sowohl im Weißlichtmodus als auch im Fluoreszenzmodus ist im Beobachtungspfad ein Beobachtungsspektralfilter angeordnet, das in den Spektralbereichen auf der kurzwelligen Seite und auf der langwelligen Seite außerhalb des Anregungsspektralbereichs durchlässig ist. Auf diese Weise wird im Weißlichtmodus ein bei der Beobachtung des Zielgebiets weitgehend natürlicher Farbeindruck gewonnen. Im Fluoreszenzmodus, in dem das Beleuchtungsspektralfilter im Beleuchtungspfad angeordnet ist, kann mit der bekannten Vorrichtung das Fluoreszenzbild mit demselben Beobachtungsspektralfilter, das auch im Weißlichtmodus verwendet wird, übertragen werden.

Bei dieser bekannten Vorrichtung ist es jedoch ebenfalls nachteilig, dass zwischen dem Fluoreszenzmodus und dem Weißlichtmodus umgeschaltet werden muss, wobei beim Umschalten das Beleuchtungsspektralfilter in den Beleuchtungspfad gebracht bzw. aus diesem entfernt werden muss.

Ein weiterer Nachteil der bekannten Vorrichtung besteht darin, dass das Beobachtungssystem nur einen Bildsensor sowohl zum Empfangen des Weißlichtbildes als auch zum Empfangen des Fluoreszenzbildes aufweist. Im Fluoreszenzmodus muss dieser Bildsensor mit erhöhter Belichtungszeit und/oder Verstärkung betrieben werden, um das meist relativ schwache Fluoreszenzsignal auf einen akzeptablen Wert zu erhöhen. Im Fluoreszenzmodus ist das dargestellte Bild daher meist stark verlangsamt und/oder verrauscht.

Es wurde auch versucht, durch Zumischen von relativ schwachem Weißlicht zu dem Fluoreszenzbild zu vermeiden, zwischen dem Weißlichtmodus und Fluoreszenzmodus ständig umschalten zu müssen. Die im Fluoreszenzbild auf diese Weise zusätzlich eingebrachte Weißlichtinformation ist jedoch aufgrund der schwachen Beleuchtung im Hinblick auf die Bildqualität von einem gewohnten Weißlichtbild weit entfernt.

Der Erfindung liegt daher die Aufgabe zugrunde, eine Vorrichtung zur Fluoreszenzdiagnose der eingangs genannten Art dahingehend weiterzubilden, dass sie gleichzeitig im Fluoreszenzmodus und Weißlichtmodus betrieben werden kann, so dass ein Umschalten zwischen beiden Betriebsarten nicht erforderlich ist, und dass dennoch ein Weißlichtbild hoher Güte erzielt wird.

Erfindungsgemäß wird diese Aufgabe hinsichtlich der eingangs genannten Vorrichtung dadurch gelöst, dass im Beleuchtungspfad sowohl im Weißlichtmodus als auch im Fluoreszenzmodus ein Beleuchtungsspektralfilter angeordnet ist, dessen Filtercharakteristik auf die Filtercharakteristik des dem zweiten Bildsensor zugeordneten Beobachtungsspektralfilter so abgestimmt ist, dass das Zielgebiet mit dem gesamten Spektralbereich des Weißlichts mit Ausnahme des typischerweise schmalen Spektralbereichs der zu beobachtenden Fluoreszenz beleuchtet wird, und im Beobachtungspfad ein Strahlteiler angeordnet ist, der den Beobachtungspfad in einen ersten Teilpfad und in einen zweiten Teilpfad aufteilt, wobei Licht entlang des ersten Teilpfads auf den ersten Bildsensor und Licht entlang des zweiten Teilpfads auf den zweiten Bildsensor gerichtet wird.

Die Bauweise und Funktion der erfindungsgemäßen Vorrichtung beruht gegenüber den bekannten Vorrichtungen auf einem neuen Konzept, das es nicht mehr erfordert, das Beobachtungsfilter in der Kamera oder im Endoskop zwischen dem Fluoreszenzmodus und dem Weißlichtmodus umzuschalten. Dies wird dadurch erreicht, dass das Zielgebiet auch im Fluoreszenzmodus mit nahezu dem gesamten Spektralbereich des von der Lichtquelle emittierten Weißlichts beleuchtet wird, wobei im Beleuchtungslicht lediglich ein schmalbandiger Spektralbereich des sichtbaren Lichts, und zwar derjenige der zu beobachtenden Fluoreszenz, fehlt. Hierzu ist im Beleuchtungspfad erfindungsgemäß ein Beleuchtungsspektralfilter angeordnet, dessen Filtercharakteristik auf die Filtercharakteristik des dem zweiten Bildsensor, der zum Empfangen des Fluoreszenzbildes dient, zugeordneten Beobachtungsspektralfilters abgestimmt ist, so dass das Zielgebiet mit dem gesamten Spektralbereich des Weißlichts mit Ausnahme des Spektralbereichs der zu beobachtenden Fluoreszenz beleuchtet wird, wobei die Filtercharakteristik des Beleuchtungsspektralfilters so gewählt ist, dass im Bereich der Fluoreszenz das Beleuchtungslicht geblockt ist. Erfindungsgemäß wird dieser Bereich möglichst eng gewählt. Das Beobachtungsspektralfilter hat vorzugsweise einen Durchlassbereich, dessen Bandbreite kleiner oder gleich 50 nm ist. Dieses Beleuchtungsspektralfilter ist sowohl im Fluoreszenzmodus als auch im Weißlichtmodus im Beleuchtungspfad angeordnet. Da nur ein schmalbandiger Spektralbereich des Weißlichts aus dem Beleuchtungslicht herausgefiltert wird, wird stets ein Weißlichtbild erhalten, das hinsichtlich Farbechtheit und Brillanz eine hohe Güte aufweist. Die erfindungsgemäße Vorrichtung ermöglicht somit die gleichzeitige Beobachtung eines Fluoreszenzbildes und Weißlichtbildes, Letzteres mit hoher Farbtreue.

Im Beobachtungspfad ist ein Strahlteiler angeordnet, der den Beobachtungspfad in einen ersten Teilpfad und in einen zweiten Teilpfad aufteilt, wobei Licht entlang des ersten Teilpfads auf den ersten Bildsensor und Licht entlang des zweiten Teilpfads auf den zweiten Bildsensor gerichtet wird.

Hierbei ist von Vorteil, dass der Beobachtungspfad, der sich beispielsweise durch ein Endoskop erstreckt, bis zum Strahlteiler ohne Aufspaltung in einzelne optische Kanäle geführt werden kann, während eine Aufteilung des Beobachtungspfads durch den Strahlteiler erst in unmittelbarer Nähe des ersten und des zweiten Bildsensors erfolgen muss. Hierdurch kann das Endoskop mit einem dünnen Schaftdurchmesser realisiert werden. Im Unterschied zu der eingangs genannten bekannten Vorrichtung kann der Strahlteiler sowohl im Weißlichtmodus als auch im Fluoreszenzmodus im Beobachtungspfad verbleiben.

Die erfindungsgemäße Vorrichtung bietet dem Benutzer eine sehr gute Orientierung im beobachteten Zielgebiet, beispielsweise einem Gewebeareal, während der Fluoreszenzdiagnose.

In einer bevorzugten Ausgestaltung ist die Filtercharakteristik des Beleuchtungsspektralfilters komplementär zur Filtercharakteristik des dem zweiten Bildsensor zugeordneten Beobachtungsspektralfilters.

Unter "komplementär" ist hier zu verstehen, dass die spektralen Filtergrenzen des Beleuchtungsspektralfilters und des dem zweiten Bildsensor zugeordneten Beobachtungsspektralfilters nahe aneinandergrenzen, vorzugsweise der Wellenlängenabstand der 50 %-Werte der Filterkurven des Beleuchtungsspektralfilters und des dem zweiten Bildsensor zugeordneten Beobachtungsspektralfilters kleiner als 15 nm, weiter vorzugsweise kleiner als 10 nm beträgt. Hierdurch wird der Vorteil erzielt, dass der aus dem Beleuchtungslicht ausgekoppelte spektrale Anteil in Abhängigkeit von der zu beobachtenden Fluoreszenz so klein wie möglich ist, wodurch die Güte des Weißlichtbildes besonders hoch ist.

In einer weiteren bevorzugten Ausgestaltung ist das dem zweiten Bildsensor zugeordnete Beobachtungsspektralfilter ein Bandpassfilter und das Beleuchtungsspektralfilter ein Kerbfilter.

In dieser Ausgestaltung liegt der schmalbandige Durchlassbereich des Beobachtungsspektralfilters mit seiner kurzwelligen Grenze und seiner langwelligen Grenze im Spektralbereich des sichtbaren Lichts.

Diese Ausgestaltung ist insbesondere in Verbindung mit der zuvor genannten Ausgestaltung, wonach die Filtercharakteristiken des Beleuchtungsspektralfilters und des dem zweiten Bildsensor zugeordneten Beobachtungsspektralfilters komplementär sind, bevorzugt.

Diese Maßnahme ist insbesondere dann vorteilhaft, wenn der verabreichte Fluoreszenzfarbstoff ein schmalbandiges Fluoreszenzspektrum aufweist, das vollständig im sichtbaren Spektralbereich liegt. In dieser Ausgestaltung bleiben dann vorteilhafterweise die Spektralbereiche beidseits des Fluoreszenzspektrums im Weißlicht erhalten, wodurch ein Weißlichtbild hoher Güte erhalten wird. Beispielsweise im Falle des Fluoreszenzfarbstoffes Fluoreszein, dessen Fluoreszenz im grünen Spektralbereich zwischen 500 und 555 nm liegt, und im Fall des Fluoreszenzfarbstoffes Protoporphyrin IX, dessen primäre Fluoreszenz im roten Spektralbereich zwischen 620 und 650 nm liegt, lässt sich diese Ausgestaltung der Filtercharakteristiken des Beleuchtungsspektralfilters und des dem zweiten Bildsensor zugeordneten Beobachtungsspektralfilters vorteilhaft nutzen. In beiden Fällen bleibt ein für ein Weißlichtbild hoher Güte ausreichender Anteil des roten Spektralbereichs im Beleuchtungslicht und somit im Weißlichtbild erhalten.

In einer weiteren bevorzugten Ausgestaltung ist dem ersten Bildsensor ein Beobachtungsspektralfilter zugeordnet, das den Spektralbereich des auf den ersten Bildsensor einfallenden Lichts auf den sichtbaren Spektralbereich begrenzt.

Hierbei ist von Vorteil, dass die Empfindlichkeit des Bildsensors zum Empfangen des Weißlichtbildes auf die typische Augenempfindlichkeit des Menschen angepasst ist, indem das vorstehend genannte Beobachtungsspektralfilter insbesondere den Infrarot-Spektralbereich blockiert. Als dem ersten Bildsensor zugeordnetes Beobachtungsspektralfilter kann beispielsweise ein BG 39-Filter der Firma Schott AG oder ein modifiziertes Filter, das noch im tiefen Rot durchlässig ist, verwendet werden.

In Zusammenhang mit dem Strahlteiler im Beobachtungspfad ist es bevorzugt, wenn das dem zweiten Bildsensor zugeordnete Beobachtungsspektralfilter in Lichtausbreitungsrichtung hinter dem Strahlteiler und vor dem zweiten Bildsensor angeordnet ist, oder dass das dem zweiten Bildsensor zugeordnete Beobachtungsspektralfilter in den Strahlteiler integriert ist.

Die zuerst genannte Alternative hat den Vorteil, herstellungstechnisch einfach zu sein, während die zweite Alternative den Vorteil hat, dass eine höhere Intensitätsausbeute sowohl für das Fluoreszenz- als auch das Weißlichtbild erhalten werden kann.

Im Zusammenhang mit der zweiten Alternative ist in einer konstruktiven Realisierung vorgesehen, dass das dem zweiten Bildsensor zugeordnete Beobachtungsspektralfilter im Spektralbereich der zu beobachtenden Fluoreszenz reflektierend und im übrigen Spektralbereich des Lichts der Weißlichtquelle transmittierend ist, oder umgekehrt.

In einer weiteren bevorzugten Ausgestaltung ist eine Steuerung für den ersten Bildsensor und/oder den zweiten Bildsensor vorhanden, die dazu ausgelegt ist, den ersten Bildsensor mit kurzer Belichtungszeit und/oder geringer Verstärkung und/oder den zweiten Bildsensor mit langer Belichtungszeit und/oder hoher Verstärkung und/oder in einem räumlichen Integrationsmodus zu betreiben.

Das Betreiben des ersten Bildsensors zum Empfangen des Weißlichtbildes mit kurzen Belichtungszeiten und niedriger Verstärkung hat den Vorteil, dass der erste Bildsensor aufgrund dessen, dass dieser relativ viel Lichtintensität empfängt, ein verzögerungsfreies rauscharmes Bild liefert. Demgegenüber hat das Betreiben des zweiten Bildsensors zum Empfangen des Fluoreszenzbildes mit langen Belichtungszeiten, hoher Verstärkung und ggf. auch mit einer räumlichen Integration den Vorteil, dass die typischerweise relativ schwache Fluoreszenz hinreichend verstärkt wird und so ein wahrnehmbares Fluoreszenzbild erhalten wird.

In diesem Zusammenhang ist es bevorzugt, wenn die Steuerung dazu ausgelegt ist, den zweiten Bildsensor in einem zeitaufgelösten Modus zu betreiben.

Diese Maßnahme ist beispielsweise bei der Untersuchung von Leckagen bei Endanastomosen, beispielsweise in der Darmchirurgie, besonders vorteilhaft, da solche Leckagen dem Arzt nach Gabe eines Fluoreszenzfarbstoffes, beispielsweise ICG, direkt visualisiert werden, und auch deren Verlauf kann zeitlich erfasst werden.

In einer weiteren bevorzugten Ausgestaltung ist der zweite Bildsensor so ausgelegt, auch in einem sogenannten Time-of-Flight-Modus betrieben zu werden.

Bei dieser Maßnahme ist die Ansteuerung des zweiten Bildsensors sowie der Lichtquelle entsprechend ausgelegt. Die vorstehend genannte Maßnahme hat den Vorteil, dass mit einem solchen Time-of-Flight-Modus die Bestimmung der ortsaufgelösten Fluoreszenz-Abklingzeit ermöglicht wird. Dies ermöglicht es prinzipiell, selbst bei gleich intensiver Fluoreszenz-Emission eines Fluoreszenzmarkers aus verschiedenen Gewebetypen eine Unterscheidung der Gewebetypen über verschiedene Abklingzeiten der Fluoreszenz zu bestimmen. Unter Fluoreszenz-Abklingzeit oder auch Fluoreszenzlebensdauer ist die mittlere Zeit zu verstehen, die nach Fluoreszenzanregung die Fluoreszenzmoleküle im angeregten Zustand verweilen, bevor ein Fluoreszenzphoton ausgesandt wird. Es handelt sich hierbei also nicht um den zeitlichen Verlauf der Fluoreszenzintensität, die beispielsweise aufgrund von Bleicheffekten oder dem Anfluten eines Fluoreszenzfarbstoffs in der Blutbahn ab- oder zunimmt.

In einer weiteren bevorzugten Ausgestaltung weist das Beobachtungssystem ein Bildverarbeitungssystem auf, das dazu ausgelegt ist, einem vom ersten Bildsensor erfassten Weißlichtbild ein vom zweiten Bildsensor erfasstes Fluoreszenzbild bzw. das Bild der ortsaufgelösten Fluoreszenz-Abklingzeit lagerichtig zu überlagern.

Hierbei ist von Vorteil, dass dem Betrachter des Fluoreszenz- und Weißlichtbildes zum einen eine räumliche Orientierung im Zielgebiet geboten wird, und zum anderen, dass die fluoreszierenden Bereiche innerhalb des Zielgebiets auch räumlich lokalisiert zugeordnet werden können, wodurch eine räumlich präzise Diagnose von malignem Gewebe ermöglicht wird.

Dabei ist es weiterhin bevorzugt, wenn das Bildverarbeitungssystem dazu ausgelegt ist, das Fluoreszenzbild in einer Falschfarbendarstellung dem Weißlichtbild zu überlagern.

Hierdurch ist von Vorteil, dass ein stärkerer Kontrast zwischen dem Weißlichtbild und dem darin überlagerten Fluoreszenzbild geschaffen wird, was die Diagnose noch präziser werden lässt. Dabei können unterschiedliche Intensitäten der Fluoreszenz durch unterschiedliche Farben visualisiert werden.

In einer weiteren bevorzugten Ausgestaltung ist das Bildverarbeitungssystem dazu ausgelegt, das Fluoreszenzbild dem Weißlichtbild nur zu überlagern, wenn das Fluoreszenzbild einen vorbestimmten Intensitätsschwellwert überschreitet, oder nur solche lokalen Bereiche des Fluoreszenzbildes dem Weißlichtbild zu überlagern, in denen der vorbestimmte Intensitätsschwellwert überschritten ist.

Diese Maßnahme trägt vorteilhafterweise zur Rauschunterdrückung des Signals des Fluoreszenzbildes bei, indem nur oberhalb eines Intensitätsschwellwertes Letzteres in das Weißlichtbild eingeblendet wird. Im Zusammenhang mit der Falschfarbendarstellung ergibt sich dabei der weitere Vorteil, dass der räumliche Intensitätsverlauf des fluoreszierenden Bereichs im Zielgebiet durch eine Farbabstufung bzw. durch die Zuordnung von unterschiedlichen Farben dargestellt werden kann, was die Diagnose noch präziser werden lässt.

Das Beobachtungssystem weist vorzugsweise ein Endoskop auf, in das der Beleuchtungspfad und/oder der Beobachtungspfad zumindest teilweise integriert ist.

Das Beobachtungssystem kann jedoch auch eine Kamera, ein Mikroskop und dgl. aufweisen.

Im Fall, dass das Beobachtungssystem ein Endoskop aufweist, kann das Endoskop ein Stereo-Endoskop sein.

Hierbei ist von Vorteil, dass mit dem Stereo-Endoskop im Weißlichtmodus ein 3D-Weißlichtbild erhalten werden kann, und im Fluoreszenzmodus kann dem 3D-Weißlichtbild das Fluoreszenzbild überlagert werden. Mittels eines Stereo-Endoskops ist insbesondere im Weißlichtmodus aufgrund der 3-Dimensionalität des Weißlichtbildes die Orientierung für den Betrachter noch weiter verbessert. Außerdem lässt sich aufgrund dessen, dass ein Stereo-Videoendoskop ohnehin bereits zwei Bildsensoren aufweist, die Erfindung in einem Stereo-Endoskop ohne großen Mehraufwand implementieren.

In einer konstruktiv besonders einfachen Ausgestaltung weist das Stereo-Endoskop zwei Optikkanäle auf, wobei in jedem der beiden Optikkanäle ein Bildsensor angeordnet ist, von denen der eine zum Empfangen eines Fluoreszenzbildes dient, wobei diesem Bildsensor das Beobachtungsspektralfilter zugeordnet ist, das in den Strahlengang eingeschwenkt werden kann, um ein Fluoreszenzbild zu erfassen, und aus dem Strahlengang herausgeschwenkt werden kann, um zusammen mit dem anderen Bildsensor ein 3D-Weißlicht zu empfangen.

In einer anderen Ausgestaltung des Stereo-Endoskops ist in beiden Optikkanälen jeweils zumindest ein Bildsensor zum Empfangen des Weißlichtbildes angeordnet, und in zumindest einem der Optikkanäle ist zumindest ein weiterer Bildsensor zum Empfangen eines Fluoreszenzbildes angeordnet. Es können aber auch in beiden Optikkanälen jeweils zusätzlich zu dem jeweiligen Bildsensor zum Empfangen eines Weißlichtbildes jeweils ein Bildsensor zum Empfangen eines Fluoreszenzbildes angeordnet sein.

Weitere Vorteile und Merkmale ergeben sich aus der nachfolgenden Beschreibung und der beigefügten Zeichnung.

Es versteht sich, dass die vorstehend genannten und nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden mit Bezug auf diese hiernach näher beschrieben. Es zeigen:
- Fig. 1: eine Vorrichtung zur Fluoreszenzdiagnose in einer schematischen Darstellung;
- Fig. 2: einen Teil eines Beobachtungssystems der Vorrichtung in Fig. 1 in einem ersten Ausführungsbeispiel;
- Fig. 3: einen Teil eines Beobachtungssystems der Vorrichtung in Fig. 1 gemäß einem weiteren Ausführungsbeispiel;
- Fig. 4: ein Diagramm einer Filtercharakteristik eines einem zweiten Bildsensor zum Empfangen eines Fluoreszenzbildes zugeordneten Beobachtungsspektralfilters und eines Beleuchtungsspektralfilters der Vorrichtung in Fig. 1 gemäß einem ersten Ausführungsbeispiel;
- Fig. 5: ein weiteres Diagramm einer Filtercharakteristik eines einem zweiten Bildsensor zum Empfangen eines Fluoreszenzbildes zugeordneten Beobachtungsspektralfilters und eines Beleuchtungsspektralfilters der Vorrichtung in Fig. 1 gemäß einem weiteren Ausführungsbeispiel;
- Fig. 6: ein noch weiteres Diagramm einer Filtercharakteristik eines einem zweiten Bildsensor zum Empfangen eines Fluoreszenzbildes zugeordneten Beobachtungsspektralfilters und eines Beleuchtungsspektralfilters der Vorrichtung in Fig. 1 gemäß einem noch weiteren Ausführungsbeispiel;
- Fig. 7: ein Ausführungsbeispiel eines in einem Stereo-Endoskop realisierten Beobachtungssystems zur Verwendung in der Vorrichtung in Fig. 1 anstelle des in Fig. 1 gezeigten Beobachtungssystems; und
- Fig. 8: ein weiteres Ausführungsbeispiel eines in einem Stereo-Endoskop realisierten Beobachtungssystems zur Verwendung in der Vorrichtung in Fig. 1 anstelle des in Fig. 1 gezeigten Beobachtungssystems.

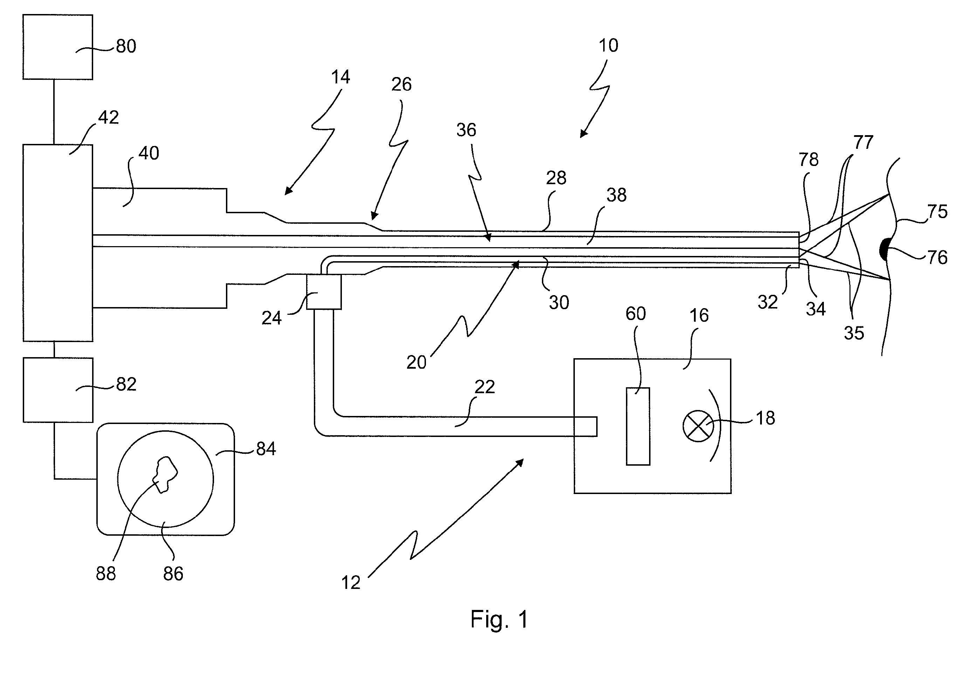

In Fig. 1 ist eine mit dem allgemeinen Bezugszeichen 10 versehene Vorrichtung zur Fluoreszenzdiagnose schematisch dargestellt. Die Vorrichtung 10 wird ohne Beschränkung der Allgemeinheit nachfolgend anhand einer Verwendung zur medizinischen Fluoreszenzdiagnose beschrieben. Die Vorrichtung 10 kann jedoch auch zur technischen Fluoreszenzdiagnose für industrielle oder wissenschaftliche Zwecke eingesetzt werden.

Die Vorrichtung 10 weist allgemein ein Beleuchtungssystem 12 und ein Beobachtungssystem 14 auf.

Das Beleuchtungssystem 12 weist eine Lichtquelle 16 auf, die dazu ausgelegt ist, Weißlicht zu erzeugen. Dazu weist die Lichtquelle 16 eine Lampe 18 oder ein Lampensystem auf, beispielsweise eine Xenon-Entladungslampe. Es können jedoch auch andere Weißlicht erzeugende Lampen oder Lampensysteme verwendet werden, wie beispielsweise Bogenentladungslampen, Glühlampen, LED-Lampensysteme, Laserdiodensysteme und dgl.

Unter "Weißlicht" im Sinne der vorliegenden Erfindung ist polychromatisches bzw. spektral breitbandiges Licht zu verstehen, das ein kontinuierliches oder quasikontinuierliches Spektrum zumindest über den sichtbaren Spektralbereich aufweist.

Das Beleuchtungssystem 12 weist weiterhin einen Beleuchtungspfad 20 auf. In dem gezeigten Ausführungsbeispiel ist der Beleuchtungspfad 20 ausgehend von der Lichtquelle 16 teilweise durch ein Lichtleitkabel 22 realisiert, das an einen Lichtleiteranschluss 24 eines Endoskops 26 angeschlossen ist. Das Endoskop 26 weist einen lang erstreckten Schaft 28 auf, durch den sich der Beleuchtungspfad 20 weiter in Form eines Lichtleiters 30, beispielsweise in Form eines Glasfaserbündels, ausgehend von dem Lichtleiteranschluss 24 zu einem distalen Ende 32 des Schafts 28 erstreckt. Über eine Lichtaustrittsfläche 34 tritt das Licht gemäß einem Lichtaustrittskegel 35 aus, wenn die Lichtquelle 16 eingeschaltet ist.

In dem gezeigten Ausführungsbeispiel ist der Beleuchtungspfad 20 somit teilweise in das Endoskop 26 integriert.

Das Beobachtungssystem 14 weist einen Beobachtungspfad 36 auf, der ebenfalls zumindest teilweise in das Endoskop 26 integriert ist. Der Beobachtungspfad 36 kann in dem Endoskop 26 auf verschiedene Weisen realisiert sein, beispielsweise durch ein geordnetes Glasfaserbündel 38 oder durch ein Relais-Linsensystem (nicht dargestellt).

Anstelle des Endoskops 26 kann die Vorrichtung 10 auch ein Mikroskop aufweisen.

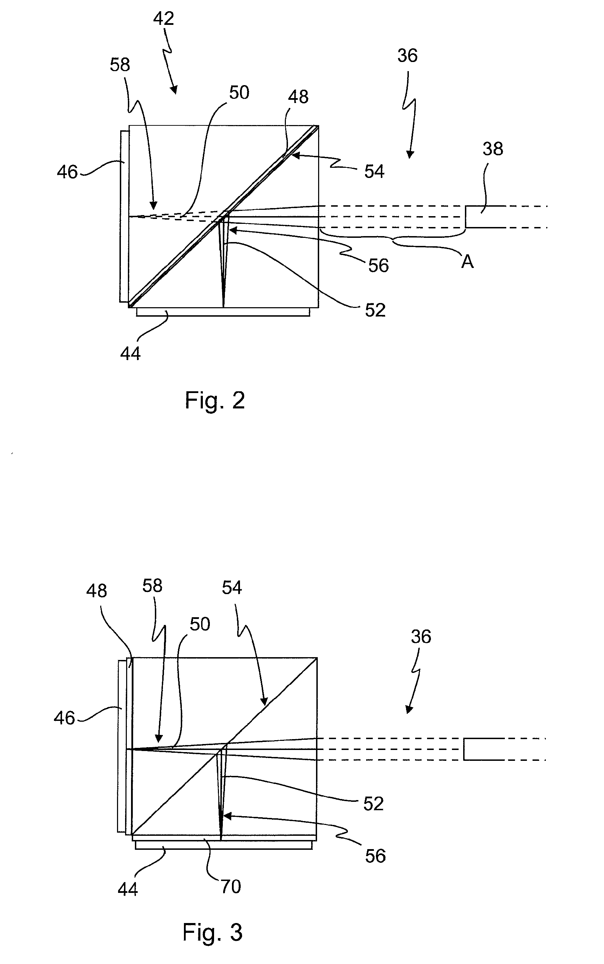

An einem proximalen Ende 40 des Endoskops 26 ist eine Kamera 42 angeschlossen, die mit Bezug auf Fig. 2 nachfolgend beschrieben wird. Es versteht sich, dass die Kamera 42 auch im Bereich des distalen Endes 32 des Schafts 28 angeordnet sein kann, so dass der Beobachtungspfad 36 entsprechend auf den distalen Bereich des Endoskops 26 beschränkt ist.

Fig. 2 zeigt die Kamera 42 des Beobachtungssystems 14 in einer schematischen vereinfachten Darstellung. Die Kamera 42, die zum Beobachtungspfad 36 gehört, weist einen ersten Bildsensor 44 und einen zweiten Bildsensor 46 auf. Es versteht sich, dass die Kamera 42 mehr als die beiden Bildsensoren 44 und 46 aufweisen kann.

Der erste Bildsensor 44 dient zum Empfangen eines Weißlichtbildes, und der zweite Bildsensor 46 dient zum Empfangen eines Fluoreszenzbildes. Dem zweiten Bildsensor 46 ist ein Beobachtungsspektralfilter 48 im Beobachtungspfad 36 zugeordnet, dessen nachfolgend noch zu beschreibende Filtercharakteristik so ausgelegt ist, dass dem zweiten Bildsensor 46 Licht im Spektralbereich zu beobachtender Fluoreszenz, d.h. Fluoreszenzlicht 50 zugespeist werden kann, während Licht 52 in den Spektralbereichen außerhalb der Fluoreszenz abgeblockt wird.

In dem Ausführungsbeispiel gemäß Fig. 2 ist das Beobachtungsspektralfilter 48, das dem zweiten Bildsensor 46 zugeordnet ist, in einen Strahlteiler 54 integriert, der den Beobachtungspfad 36 in einen ersten Teilpfad 56 und einen zweiten Teilpfad 58 aufteilt. Das Licht 52 wird somit entlang des ersten Teilpfads 56 auf den ersten Bildsensor 44 gerichtet, und das Licht 50 (Fluoreszenzlicht) wird auf den zweiten Bildsensor 46 entlang des zweiten Teilpfads 58 gerichtet.

Bei dem Ausführungsbeispiel gemäß Fig. 2 ist das Beobachtungsspektralfilter 48 im Spektralbereich der zu beobachtenden Fluoreszenz durchlässig und in den übrigen Spektralbereichen außerhalb der Fluoreszenz reflektierend.

Ein Objektiv, das üblicherweise den Bildsensoren 44 und 46 vorgeschaltet ist, und das beispielsweise in dem Bereich A in Fig. 2 angeordnet ist, ist in Fig. 2 der Übersichtlichkeit halber weggelassen worden.

Alternativ zur Integration des dem zweiten Bildsensor 46 zugeordneten Beobachtungsspektralfilters 48 kann Letzteres auch zwischen dem Strahlteiler 54 und dem zweiten Bildsensor 46 angeordnet sein, beispielsweise unmittelbar vor dem zweiten Bildsensor 46, so dass das Licht 50 im Bereich zwischen dem Strahlteiler 54 und dem Beobachtungsspektralfilter 48 nicht nur den Spektralanteil der Fluoreszenz enthält, sondern auch die Spektralanteile außerhalb der Fluoreszenz. Das Gleiche gilt für die spektrale Zusammensetzung des Lichts 52 im ersten Teilpfad 56. Dies ist in Fig. 3 gezeigt.

Wieder mit Bezug auf Fig. 1 weist der Beleuchtungspfad 20 ein Beleuchtungsspektralfilter 60 auf, dessen Filtercharakteristik auf die Filtercharakteristik des dem zweiten Bildsensor 46 zugeordneten Beobachtungsspektralfilters 48 so abgestimmt ist, dass das Beleuchtungslicht den gesamten Spektralbereich des von der Lichtquelle 16 erzeugten Weißlichts mit Ausnahme des Spektralbereichs der zu beobachtenden Fluoreszenz enthält. Das Beleuchtungsspektralfilter 60 ist in Fig. 1 in der Lichtquelle 16 angeordnet, kann jedoch auch an anderer Stelle im Beleuchtungspfad 22 vor dem Austritt des Lichts aus dem distalen Ende 32 angeordnet sein.

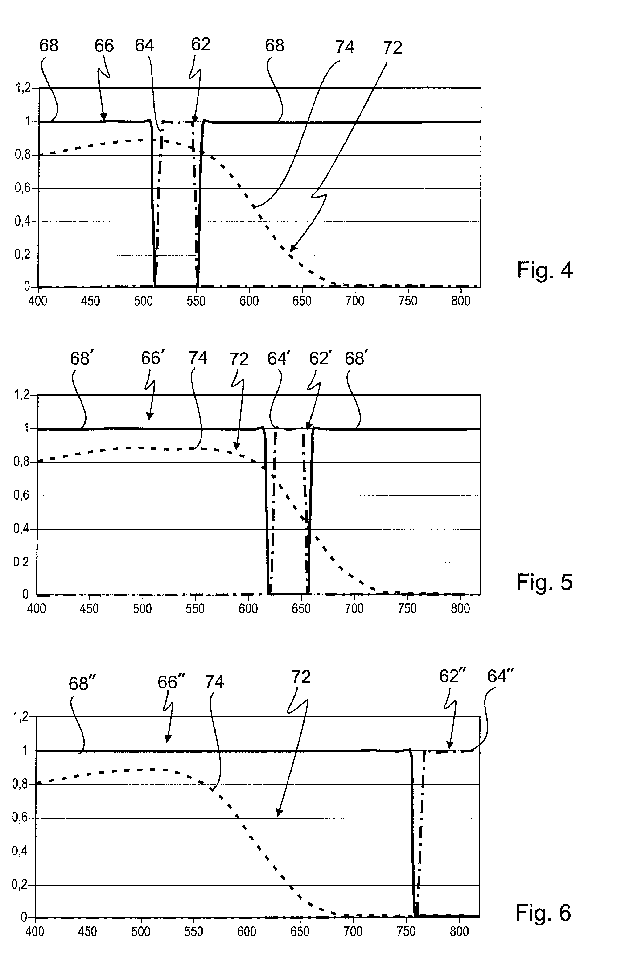

In Fig. 4 bis 6 sind Ausführungsbeispiele für die Filtercharakteristiken des Beobachtungsspektralfilters 48 und des Beleuchtungsspektralfilters 60 gezeigt.

In Fig. 4 ist eine Filtercharakteristik 62 des dem zweiten Bildsensor 46 zugeordneten Beobachtungsspektralfilters 48 gezeigt, gemäß der das Beobachtungsspektralfilter 48 als Bandpassfilter ausgebildet ist. Eine Filterkurve 64, die den Verlauf der Transmission des Beobachtungsspektralfilters 48 wiedergibt, zeigt, dass der Durchlassbereich des Beobachtungsspektralfilters 48 sehr schmalbandig ist und im Beispielfall auf den Spektralbereich von etwa 510 bis 550 nm beschränkt ist. Diese Ausführungsform eignet sich insbesondere für den Fluoreszenzfarbstoff Fluoreszein, dessen Fluoreszenz in diesem Spektralbereich liegt.

Eine Filtercharakteristik 66 des Beleuchtungsspektralfilters 60 ist durch eine Filterkurve 68 dargestellt, gemäß der das Beleuchtungsspektralfilter 60 ein Kerbfilter ("notch-filter") ist. Das Beleuchtungsspektralfilter 60 ist gemäß der Filterkurve 68, wie in Fig. 4 gezeigt ist, in allen Spektralbereichen des sichtbaren Lichts, mit Ausnahme des Spektralbereichs, in dem das Beobachtungsspektralfilter 48 durchlässig ist, durchlässig. Im Spektralbereich der zu beobachtenden Fluoreszenz, in dem das Beobachtungsspektralfilter 48 durchlässig ist, ist das Beleuchtungsspektralfilter 60 hingegen undurchlässig.

In Fig. 5 ist ein weiteres Ausführungsbeispiel für die Filtercharakteristiken 62' des Beobachtungsspektralfilters 48 und 66' des Beleuchtungsspektralfilters 60 gezeigt. Bei diesem Ausführungsbeispiel ist das Beobachtungsspektralfilter 48 ebenfalls als Bandpassfilter ausgebildet, wobei das Beobachtungsspektralfilter 48 in einem Spektralbereich von etwa 620 nm bis etwa 655 nm durchlässig ist. Die Filtercharakteristik 66' des Beleuchtungsspektralfilters 60 weist eine Filterkurve 68' auf, gemäß der das Beleuchtungsspektralfilter 60 als Kerbfilter ausgebildet ist, dessen Durchlässigkeit komplementär zur Durchlässigkeit des Beobachtungsspektralfilters 48 ist. Diese Filtercharakteristika 62' und 66' eignen sich für den Fluoreszenzfarbstoff Protoporphyrin IX, dessen Fluoreszenzmaximum im Spektralbereich von etwa 620 nm bis etwa 655 nm liegt.

Fig. 6 zeigt ein Ausführungsbeispiel für Filtercharakteristiken 62" und 66" des Beobachtungsspektralfilters 48 und des Beleuchtungsspektralfilters 60, bei dem das Beobachtungsspektralfilter 48 als Langpassfilter und das Beleuchtungsspektralfilter 60 als Kurzpassfilter ausgebildet ist, wobei das kurzwellige Ende der Filterkurve 64" und das langwellige Ende der Filterkurve 68" bei einer Wellenlänge von etwa 760 nm liegen. Die Filtercharakteristiken 62" und 68" eignen sich insbesondere für den Fall, dass eine Fluoreszenzdiagnose mittels des Fluoreszenzfarbstoffes ICG (Indocyaningrün) durchgeführt wird.

In allen Ausführungsbeispielen gemäß Fig. 4 bis 6 sind die Filtercharakteristiken 62, 66 bzw. 62', 66' bzw. 62", 66" des Beobachtungsspektralfilters 48 und des Beleuchtungsspektralfilters 60 komplementär zueinander, d.h. die einander benachbarten Grenzen der Durchlassbereiche des Beleuchtungsspektralfilters 60 und des Beobachtungsspektralfilters 48 liegen in einem Wellenlängenabstand von weniger als 15 nm voneinander, bezogen auf den 50 %-Transmissionswert der Filterkurven 64, 68 bzw. 64', 68' bzw. 64", 68".

Gemäß Fig. 3 ist dem ersten Bildsensor 44 ein weiteres Beobachtungsspektralfilter 70 zugeordnet, das den Spektralbereich des auf den ersten Bildsensor 44 einfallenden Lichts auf den sichtbaren Spektralbereich begrenzt. Ein solches weiteres Beobachtungsspektralfilter 70 kann ein BG 39-Filter der Firma Schott AG oder ein anderes, gegenüber dem BG 39-Filter undefiniertes Filter sein. In Fig. 4 bis 6 ist jeweils die Filtercharakteristik 72 dieses weiteren Beobachtungsspektralfilters 70 durch eine Filterkurve 74 dargestellt. Das weitere Beobachtungsspektralfilter 70 verringert insbesondere Spektralanteile des Lichts im fernen roten Bereich des sichtbaren Spektrums.

Mit Bezug auf Fig. 1 werden nachfolgend weitere Einzelheiten der Vorrichtung 10 und deren Funktionsweise näher beschrieben.

Die Vorrichtung 10 wird in einer Fluoreszenzdiagnose gleichzeitig im Weißlichtmodus und im Fluoreszenzmodus betrieben. Sowohl im Weißlichtmodus als auch im Fluoreszenzmodus ist das Beleuchtungsspektralfilter 60 im Beleuchtungspfad 20 vorhanden. Ebenso ist das Beobachtungsspektralfilter 48 sowohl im Weißlichtmodus als auch im Fluoreszenzmodus im Beobachtungspfad 36 angeordnet.

Von der Lichtquelle 16 erzeugtes Weißlicht wird durch das Beleuchtungsspektralfilter 60 in das Lichtleitkabel 22 eingespeist und über den Lichtleiter 30 zum distalen Ende 32 des Schafts 28 des Endoskops 26 geleitet, wo es austritt und ein Zielgebiet 75 gemäß dem Lichtaustrittskegel 35 beleuchtet. Das Zielgebiet 75 ist beispielsweise ein Gewebeareal im menschlichen oder tierischen Körper, das auf das Vorhandensein von malignem Gewebe hin untersucht werden soll. Durch das Beleuchtungslicht wird eine im Zielgebiet 75 befindliche fluoreszierende Substanz 76 zur Fluoreszenz angeregt. Die fluoreszierende Substanz 76 kann beispielsweise Fluoreszein, Protoporphyrin oder Indocyaningrün sein, wobei die Filtercharakteristiken des Beleuchtungsspektralfilters 60 und des Beobachtungsspektralfilters 48 gemäß den Fig. 4 bis 6 entsprechend auf den jeweiligen Fluoreszenzfarbstoff ausgelegt sind.

Das vom Fluoreszenzfarbstoff 76 emittierte Fluoreszenzlicht sowie das vom Zielgebiet 75 reflektierte Beleuchtungslicht werden gemäß einem Beobachtungslichtkegel 77 vom distalen Ende 32 des Schafts 28 des Endoskops 36 empfangen und treten in das distale Ende 78 des Beobachtungspfades 36, hier des Glasfaserbündels 38 ein. Das Beobachtungslicht wird dann nach proximal geleitet, wobei nur das Fluoreszenzlicht durch das Beobachtungsspektralfilter 48 auf den zweiten Bildsensor 46 und das rückreflektierte Beleuchtungslicht (Weißlicht) auf den ersten Bildsensor 44 trifft.

Die Vorrichtung 10 weist eine Steuerung 80 für den ersten Bildsensor 44 und für den zweiten Bildsensor 46 auf, die den ersten Bildsensor 44 mit kurzer Belichtungszeit und/oder geringer Verstärkung betreibt, um so ein Weißlichtbild mit hoher Bildqualität zu erhalten.

Die Steuerung 80 betreibt dagegen den zweiten Bildsensor 46 mit langer Belichtungszeit und/oder hoher Verstärkung und/oder in einem räumlichen Integrationsmodus, um möglichst viel von dem intensitätsschwachen Fluoreszenzlicht aufzunehmen.

Die Steuerung 80 und der zweite Bildsensor 46 sind insbesondere dazu in der Lage, mittels eines Time-of-Flight-Modus eine ortsaufgelöste Bestimmung der Fluoreszenz-Abklingzeit durchzuführen.

Die von den Bildsensoren 44 und 46 gelieferten Bildsignale werden dann einem Bildverarbeitungssystem 82 zugeführt, das die empfangenen Bildsignale verarbeitet und einem Bildwiedergabegerät 84, beispielsweise einem Farbmonitor zuführt.

Das Bildverarbeitungssystem 82 ist dabei in der Lage, einem von dem ersten Bildsensor 44 erfassten Weißlichtbild 86 ein von dem zweiten Bildsensor 46 erfasstes Fluoreszenzbild 88 lagerichtig zu überlagern, d.h. das Fluoreszenzbild 88 lagerichtig in das Weißlichtbild 86 einzublenden. Der Benutzer kann somit aufgrund des Fluoreszenzbildes 88 nicht nur erkennen, dass im Zielgebiet 75 malignes Gewebe vorhanden ist, sondern er kann auch genau orten, wo im Zielgebiet 75 das maligne Gewebe vorhanden ist.

Das Fluoreszenzbild 88 wird dabei dem Weißlichtbild 86 in einer Falschfarbendarstellung überlagert, wodurch der Kontrast zwischen dem Fluoreszenzbild und dem Weißlichtbild 86 verstärkt werden kann. Dabei kann außerdem die Falschfarbendarstellung für unterschiedliche Intensitäten des Fluoreszenzlichts unterschiedliche Farben bereitstellen, um so den Intensitätsverlauf des Fluoreszenzbildes 88 zu visualisieren.

Außerdem kann das Bildverarbeitungssystem 82 in der Lage sein, das Fluoreszenzbild 88 dem Weißlichtbild 86 nur zu überlagern, wenn das Fluoreszenzbild 88 einen vorbestimmten Intensitätsschwellwert überschreitet, bzw. von dem gesamten Areal im Zielgebiet 75, aus dem Fluoreszenzlicht vom zweiten Bildsensor 46 empfangen wird, werden nur solche lokalen Bereiche des Fluoreszenzbildes 88 dem Weißlichtbild 86 überlagert, in denen der vorbestimmte Intensitätsschwellwert überschritten ist.

Die Vorrichtung 10 ist, wie aus der vorhergehenden Beschreibung hervorgeht, in der Lage, ohne Umschalten zwischen dem Weißlichtmodus und dem Fluoreszenzmodus das Weißlichtbild 86 mit sehr hoher Bildqualität, insbesondere hoher Farbtreue auf dem Bildwiedergabegerät 84 darzustellen, und gleichzeitig das Fluoreszenzbild 88 in das Weißlichtbild 86 mit hohem Kontrast zum Weißlichtbild 86 einzublenden.

Wenn der zweite Bildsensor 46 in einem Time-of-Flight-Modus betrieben wird, kann die ortsaufgelöste Fluoreszenz-Abklingzeit visualisiert werden. Die Visualisierung kann beispielsweise über ein Grauskalenbild oder eine Codierung mittels Falschfarben realisiert sein.

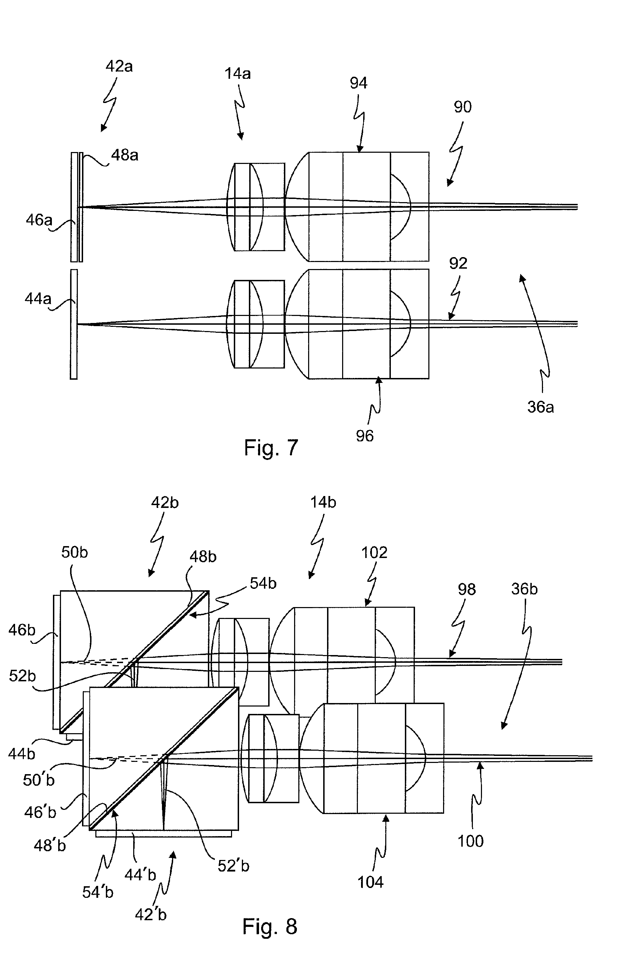

Während das Beobachtungssystem 14 der Vorrichtung 10 gemäß Fig. 1 einen Beobachtungspfad 36 aufweist, der optisch einkanalig ist, bzw. das Endoskop 26 als Mono-Endoskop ausgeführt ist, zeigen Fig. 7 und 8 zwei Ausführungsbeispiele, bei der die vorliegende Erfindung auch in Beobachtungssystemen mit zweikanaliger Optik, d.h. mit einem Stereo-Endoskop bzw. Stereo-Videoendoskop realisiert sein kann.

Fig. 7 zeigt ein erstes Ausführungsbeispiel einer solchen Realisierung eines Beobachtungssystems 14a, das Bestandteil eines Stereo-Videoendoskops ist. Teile des Beobachtungssystems 14a, die mit Teilen des Beobachtungssystems 14 gleich oder vergleichbar sind, sind mit den gleichen Bezugszeichen versehen wie die Teile des Beobachtungssystems 14, ergänzt um ein "a".

Ein solches Stereo-Endoskop wird dann in der Vorrichtung 10 anstelle des Endoskops 26 verwendet.

Der Beobachtungspfad 36a des Beobachtungssystems 14a ist optisch zweikanalig aufgebaut, mit einem ersten Optikkanal 90 und einem zweiten Optikkanal 92. Das Beobachtungssystem 14a weist zwei Bildsensoren 44a und 46a auf, wie dies bei Stereo-Videoendoskopen üblich ist. Dem Bildsensor 46a ist ein erstes Objektiv 94 zugeordnet, und dem Bildsensor 44a ist ein zweites Objektiv 96 zugeordnet. Bei dieser beispielhaften Ausführung weisen die beiden Optikkanäle 90 und 92 in Bezug auf das zu beobachtende Zielgebiet, hier das Zielgebiet 75 gemäß Fig. 1 einen Winkel ≠ 0° zueinander auf, wie dies den Blickrichtungen der beiden Augen eines Menschen entspricht.

Der erste Bildsensor 44a dient bei dem Beobachtungssystem 14a zum Empfangen eines Weißlichtbildes, und der zweite Bildsensor 46a dient zum Empfangen eines Fluoreszenzbildes. Dem zweiten Bildsensor 46a ist entsprechend ein Beobachtungsspektralfilter 48a zugeordnet, dessen Filtercharakteristik beispielsweise die Filtercharakteristik 62 in Fig. 4, die Filtercharakteristik 62' in Fig. 5 oder die Filtercharakteristik 62" in Fig. 6 ist. Das Beleuchtungsspektralfilter 60 gemäß Fig. 1 ist entsprechend an die Filtercharakteristik des Beobachtungsspektralfilters 48a angepasst.

Bei dem in Fig. 7 gezeigten Ausführungsbeispiel kann das Beobachtungsspektralfilter 48a aus dem Strahlengang herausgeschwenkt werden, so dass mit den beiden Bildsensoren 46a und 44a ein Weißlichtbild mit stereoskopischem Effekt, d.h. ein 3D-Weißlichtbild erhalten wird. Im Fluoreszenzmodus ist das Beobachtungsspektralfilter 48a in den Strahlengang eingeschwenkt, und es kann bei dem Beobachtungssystem 14 ein Weißlichtbild mit darin eingeblendetem Fluoreszenzbild visualisiert werden.

Die Ausführungsform des Beobachtungssystems 14a, die konstruktiv sehr einfach ist, wird vorzugsweise dann in Betracht gezogen, wenn das zu beobachtende Zielgebiet 75 hinreichend weit von den Objektiven 94 und 96 beabstandet ist, um im Fluoreszenzmodus eine lagerichtige Überlagerung des Fluoreszenzbildes im Weißlichtbild zu erhalten.

Fig. 8 zeigt eine gegenüber Fig. 7 weiterentwickelte Ausführungsform eines Beobachtungssystems 14b, das ebenfalls als Stereo-Videoendoskop-Beobachtungssystem realisiert ist, und das sich auch für kleine Entfernungen zum Zielgebiet 72 eignet.

Der Beobachtungspfad 36b ist wiederum zweikanalig mit einem ersten Optikkanal 98 und einem zweiten Optikkanal 100 ausgestaltet. Im ersten Optikkanal 98 ist ein Objektiv 102 und im zweiten Optikkanal 100 ist ein Objektiv 104 angeordnet.

Das Beobachtungssystem 14b unterscheidet sich von dem Beobachtungssystem 14a hinsichtlich der Anzahl und Anordnung der Bildsensoren.

Bei dieser Ausführungsform ist der erste Optikkanal 98 mit einer Kamera 42b ausgestattet, die mit der Kamera 42 in Fig. 2 identisch ist. Die Kamera 42b weist entsprechend einen ersten Bildsensor 44b zum Empfangen eines Weißlichtbildes und einen zweiten Bildsensor 46b zum Empfangen eines Fluoreszenzbildes, ein dem zweiten Bildsensor 46b zugeordnetes Beobachtungsspektralfilter 48b und einen Strahlteiler 54b auf.

Der zweite Optikkanal 100 weist eine dazu identische Kamera 42'b auf, mit einem ersten Bildsensor 44'b zum Empfangen eines Weißlichtbildes, einem zweiten Bildsensor 46'b zum Empfangen eines Fluoreszenzbildes, ein dem zweiten Bildsensor 46'b zugeordnetes Beobachtungsspektralfilter 48'b und einen Strahlteiler 54'b auf.

In dieser Ausgestaltung ist es möglich, gleichzeitig ein 3D-Weißlichtbild und darin eine Überlagerung eines Fluoreszenzbildes zu visualisieren.

Eine gegenüber dem Beobachtungssystem 14b vereinfachte Ausführungsform kann darin bestehen, dass bei einer der beiden Kameras 42b oder 42'b der zweite Bildsensor 46b oder 46'b weggelassen wird (und der entsprechende Strahlteiler 54b bzw. 54'b dann weggelassen oder voll reflektierend ist), wobei dann das Fluoreszenzbild nicht stereoskopisch erfasst wird.

## Patentansprüche

1. Vorrichtung zur Fluoreszenzdiagnose, mit einem Beleuchtungssystem (12) zum Beleuchten eines Zielgebiets (75), das eine Lichtquelle (16), die dazu ausgelegt ist, Weißlicht zu erzeugen, und einen Beleuchtungspfad (20) aufweist, und mit einem Beobachtungssystem (14; 14a; 14b) zum Beobachten des Zielgebiets (75) in einem Weißlichtmodus und in einem Fluoreszenzmodus, wobei das Beobachtungssystem (14; 14a; 14b) einen Beobachtungspfad (36; 36a; 36b) aufweist, der zumindest einen ersten Bildsensor (44; 44a; 44b, 44'b) zum Empfangen eines Weißlichtbildes und zumindest einen zweiten Bildsensor (46; 46a; 46b, 46'b) zum Empfangen eines Fluoreszenzbildes aufweist, wobei dem zweiten Bildsensor (46; 46a; 46b, 46'b) im Beobachtungspfad (36; 36a; 36b) ein Beobachtungsspektralfilter (48; 48a; 48b, 48'b) zugeordnet ist, dessen Filtercharakteristik (62; 62'; 62") so ausgelegt ist, dass dem zweiten Bildsensor (46; 46a; 46b, 46'b) Licht im Spektralbereich zu beobachtender Fluoreszenz zugespeist werden kann, während Licht in den Spektralbereichen außerhalb der Fluoreszenz abgeblockt wird, **dadurch gekennzeichnet, dass** im Beleuchtungspfad (20) sowohl im Weißlichtmodus als auch im Fluoreszenzmodus ein Beleuchtungsspektralfilter (60) angeordnet ist, dessen Filtercharakteristik (66; 66'; 66") auf die Filtercharakteristik (62; 62'; 62") des dem zweiten Bildsensor (46; 46a; 46b, 46'b) zugeordneten Beobachtungsspektralfilters (48; 48a; 48b, 48'b) so abgestimmt ist, dass das Zielgebiet (75) mit dem gesamten Spektralbereich des Weißlichts mit Ausnahme des Spektralbereichs der zu beobachtenden Fluoreszenz beleuchtet wird, und im Beobachtungspfad (36; 36b) ein Strahlteiler (54; 54b, 54'b) angeordnet ist, der den Beobachtungspfad (36; 36b) in einen ersten Teilpfad (56; 56b, 56'b) und in einen zweiten Teilpfad (58; 58b, 58'b) aufteilt, wobei Licht entlang des ersten Teilpfads (56; 56b, 56'b) auf den ersten Bildsensor (44; 44b, 44'b) und Licht entlang des zweiten Teilpfads (58; 58b, 58'b) auf den zweiten Bildsensor (46; 46b, 46'b) gerichtet wird.

2. Vorrichtung nach Anspruch 1, **dadurch gekennzeichnet, dass** die Filtercharakteristik (66; 66'; 66") des Beleuchtungsspektralfilters (60) komplementär zur Filtercharakteristik (62; 62'; 62") des dem zweiten Bildsensor (46; 46a; 46b, 46'b) zugeordneten Beobachtungsspektralfilters (48; 48a; 48b, 48'b) ist.

3. Vorrichtung nach Anspruch 1 oder 2, **dadurch gekennzeichnet, dass** das dem zweiten Bildsensor (46; 46a; 46b, 46'b) zugeordnete Beobachtungsspektralfilter (48; 48a; 48b, 48'b) ein Bandpassfilter und das Beleuchtungsspektralfilter (60) ein Kerbfilter ist.

4. Vorrichtung nach einem der Ansprüche 1 bis 3, **dadurch gekennzeichnet, dass** dem ersten Bildsensor (44) ein Beobachtungsspektralfilter (70) zugeordnet ist, das den Spektralbereich des auf den ersten Bildsensor (44) einfallenden Lichts auf den sichtbaren Spektralbereich begrenzt.

5. Vorrichtung nach einem der Ansprüche 1 bis 4, **dadurch gekennzeichnet, dass** das dem zweiten Bildsensor (46) zugeordnete Beobachtungsspektralfilter (48) in Lichtausbreitungsrichtung hinter dem Strahlteiler (54) und vor dem zweiten Bildsensor (46) angeordnet ist.

6. Vorrichtung nach einem der Ansprüche 1 bis 4, **dadurch gekennzeichnet, dass** das dem zweiten Bildsensor (46; 46b, 46'b) zugeordnete Beobachtungsspektralfilter (48; 48b, 48'b) in den Strahlteiler integriert ist.

7. Vorrichtung nach Anspruch 6, **dadurch gekennzeichnet, dass** das dem zweiten Bildsensor (46; 46b, 46'b) zugeordnete Beobachtungsspektralfilter (48; 48b, 48'b) im Spektralbereich der zu beobachtenden Fluoreszenz reflektierend und im übrigen Spektralbereich des Lichts der Weißlichtquelle transmittierend ist oder umgekehrt.

8. Vorrichtung nach einem der Ansprüche 1 bis 7, **dadurch gekennzeichnet, dass** eine Steuerung (80) für den ersten Bildsensor (44; 44a; 44b, 44'b) und/oder den zweiten Bildsensor (46; 46a; 46b, 46'b) vorhanden ist, die dazu ausgelegt ist, den ersten Bildsensor (44; 44a; 44b, 44'b) mit kurzer Belichtungszeit und/oder geringer Verstärkung und/oder den zweiten Bildsensor (46; 46a; 46b, 46'b) mit langer Belichtungszeit und/oder hoher Verstärkung und/oder in einem räumlichen Integrationsmodus zu betreiben.

9. Vorrichtung nach Anspruch 8, **dadurch gekennzeichnet, dass** die Steuerung (80) dazu ausgelegt ist, den zweiten Bildsensor (46; 46a; 46b, 46'b) in einem zeitaufgelösten Modus zu betreiben.

10. Vorrichtung nach Anspruch 8 oder 9, **dadurch gekennzeichnet, dass** die Steuerung (80) und der zweite Bildsensor (46; 46a; 46b, 46b') dazu ausgelegt sind, die Fluoreszenz-Abklingzeit ortsaufgelöst zu bestimmen.

11. Vorrichtung nach einem der Ansprüche 1 bis 10, **dadurch gekennzeichnet, dass** das Beobachtungssystem (14; 14a; 14b) ein Bildverarbeitungssystem (84) aufweist, das dazu ausgelegt ist, einem vom ersten Bildsensor (44; 44a; 44b, 44'b) erfassten Weißlichtbild (86) ein vom zweiten Bildsensor (46; 46a; 46b, 46'b) erfasstes Fluoreszenzbild (88) bzw. das Bild der ortsaufgelösten Fluoreszenz-Abklingzeit lagerichtig zu überlagern.

12. Vorrichtung nach Anspruch 11, **dadurch gekennzeichnet, dass** das Bildverarbeitungssystem (82) dazu ausgelegt ist, das Fluoreszenzbild (88) in einer Falschfarbendarstellung dem Weißlichtbild (86) zu überlagern.

13. Vorrichtung nach Anspruch 11 oder 12, **dadurch gekennzeichnet, dass** das Bildverarbeitungssystem (82) dazu ausgelegt ist, das Fluoreszenzbild (88) dem Weißlichtbild (86) nur zu überlagern, wenn das Fluoreszenzbild (88) einen vorbestimmten Intensitätsschwellwert überschreitet, oder nur solche lokalen Bereiche des Fluoreszenzbildes (88) dem Weißlichtbild (86) zu überlagern, in denen der vorbestimmte Intensitätsschwellwert überschritten ist.

14. Vorrichtung nach einem der Ansprüche 1 bis 13, **dadurch gekennzeichnet, dass** das Beobachtungssystem (14; 14a; 14b) ein Endoskop (26) aufweist, in das der Beleuchtungspfad (20) und/oder der Beobachtungspfad (36; 36a; 36b) zumindest teilweise integriert ist.

15. Vorrichtung nach Anspruch 14, **dadurch gekennzeichnet, dass** das Endoskop ein Stereo-Endoskop ist.
